# EUROPEAN PATENT APPLICATION

(11) **EP 4 617 005 A1**
(43) Date of publication of application: **17.09.2025**
(21) Application number: 23888783.0
(22) Date of filing: 09.11.2023
(51) Int. Cl.: B25J 5/00, B25J 15/10, C12M 1/00, C12M 1/26

(54) **ROBOT SYSTEM**

(30) Priority: 09.11.2022 JP 2022179647
(71) Applicant: OMRON Corporation, Kyoto 600-8530 (JP); CHUGAI SEIYAKU KABUSHIKI KAISHA, Tokyo 115-8543 (JP)
(72) Inventor: NAKASHIMA, Chisato, Kyoto-shi, Kyoto 600-8530 (JP); HAYASHI, Kennosuke, Kyoto-shi, Kyoto 600-8530 (JP); NABETO, Misato, Kyoto-shi, Kyoto 600-8530 (JP); SHIBATA, Yoshiya, Kyoto-shi, Kyoto 600-8530 (JP); OKI, Kentaro, Kyoto-shi, Kyoto 600-8530 (JP); ABE, Masayoshi, Kyoto-shi, Kyoto 600-8530 (JP); SUGIHARA, Tomomichi, Kyoto-shi, Kyoto 600-8530 (JP)
(74) Representative: SONN Patentanwälte GmbH & Co KG
(86) International application number: PCT/JP2023/040472
(87) International publication number: WO 2024/101438

(57) **Abstract**

A robot system (100) includes a workbench robot (20) including a manipulator that manipulates an object on a workbench, and a controller (30) that controls an operation of the workbench robot (20), the manipulator including an articulated arm and a hand that is positioned at the distal end of the arm and includes plural fingers having j oints, the hand having a structure in which a first group of fingers including a first finger and a second group of fingers including a second finger and a third finger face each other and hold the object therebetween, the controller (30) causing the workbench robot (20) to perform at least two kinds of processes among the processes of causing the hand to hold an aspirator to aspirate a liquid, causing the hand to hold an electric pipettor to suck and discharge the liquid, causing the hand to hold an electric micropipette to suck and discharge the liquid, and causing the hand to hold a spatula to scoop and transfer a powder material.

## Description

### Technical Field

The present disclosure relates to a robot system.

### Background Art

In automating experiments for handling biological samples such as cells, various attempts have been made to cause robots to handle tools of the same type as those usually used by researchers.

For example, a technique for causing a robot to handle an electric pipette (Patent Literature 1), and a technique for causing a robot to handle various kinds of containers (Patent Literature 2) have been proposed.

### Citation List

### Patent Literatures

Patent Literature 1: Japanese Patent Publication No. 6735475
Patent Literature 2: Japanese Patent Publication No. 5776772

### SUMMARY OF INVENTION

### Technical Problem

As shown in Fig. 2 of Patent Literature 1, a hand of a robot disclosed in Patent Literature 1 includes two rod-like fingers called claws, and holds a pipette device shown in Fig. 3 of Patent Literature 1 with the two claws. Also, the robot disclosed in Patent Literature 1 is described as if it handled a pipette device for a measuring pipette in Fig. 18 of Patent Literature 1.

However, since it is obvious that the operation of pressing the suction button and the discharge button while holding the pipette device for a measuring pipette with the hand disclosed in Patent Literature 1 cannot be performed, handling the pipette device for a measuring pipette is merely a wish.

A hand of a robot disclosed in Patent Literature 2 includes two plate-like fingers called bits. Each bit has several recesses and pins (grip members 73) projecting perpendicularly from a plate face so as to conform to specific shapes of several types of containers intended to be handled. As described above, in a case where the operation target cannot be handled with fingers having a simple shape, the shape of the fingers is specialized in accordance with the operation target.

However, if hands are specialized in accordance with the operation target, the hands needs to be replaced every time the operation target changes. For example, it is obvious that the hand disclosed in Patent Literature 2 specialized for holding a specific container is not suitable for holding a pipette device. When a robot attempts to perform an experiment handling a biological sample such as cells in the same procedures as those used by a researcher, it is necessary to perform a series of works while sequentially using a plurality of types of tools on one workbench, but the necessity of replacement of the hand in the middle hinders automation of the experiment.

The present disclosure has been made in view of the above aspects, and aims to increase the number of types of work that can be performed with one type of hand in a robot system that performs a series of works on a workbench that requires handling of a plurality of types of tools, and is used in the fields of experiments of handling biological samples such as cells.

### Solution to Problem

To achieve the above objective, a robot system according to the present disclosure is a robot system that includes a workbench robot including a manipulator that manipulates an object on a workbench, and a controller that controls operations of the workbench robot. In the robot system, the manipulator includes an articulated arm, and a hand that is positioned at the distal end of the arm and that includes a plurality of fingers having joints. The hand has a structure in which a first group of fingers including a first finger and a second group of fingers including a second finger and a third finger face each other and hold the object therebetween. The controller causes the workbench robot to perform at least two kinds of processes among a process of causing the hand to hold an aspirator to aspirate a liquid, a process of causing the hand to hold an electric pipettor to suck the liquid and a process of causing the electric pipettor to discharge the liquid, a process of causing the hand to hold an electric micropipette to suck the liquid and a process of causing the electric micropipette to discharge the liquid, or a process of causing the hand to hold a spatula to scoop and transfer a powder material.

### Advantageous Effects of Invention

With a robot system according to the present disclosure, it is possible to increase the number of types of work that can be performed with one type of hand in the robot system that performs a series of works on a workbench that requires handling of a plurality of types of tools, and is used in the fields of experiments of handling biological samples such as cells.

### BRIEF DESCRIPTION OF DRAWINGS

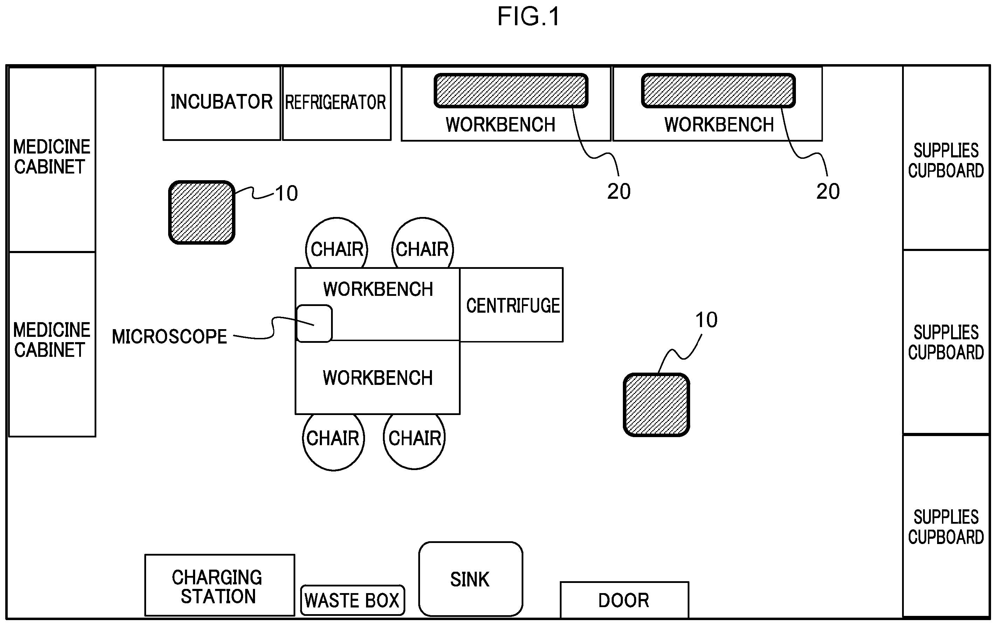

Fig. 1 is a schematic plan view of an example of an experimental environment in which a robot system according to the present embodiment operates.

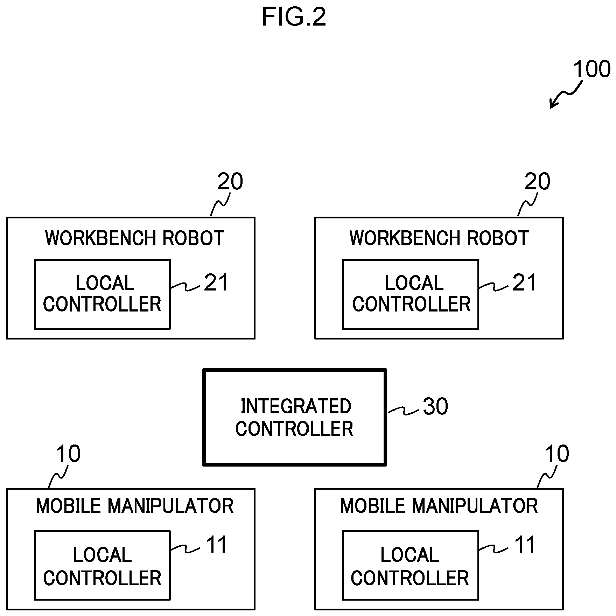

Fig. 2 is a diagram showing a schematic configuration of the robot system according to the embodiment.

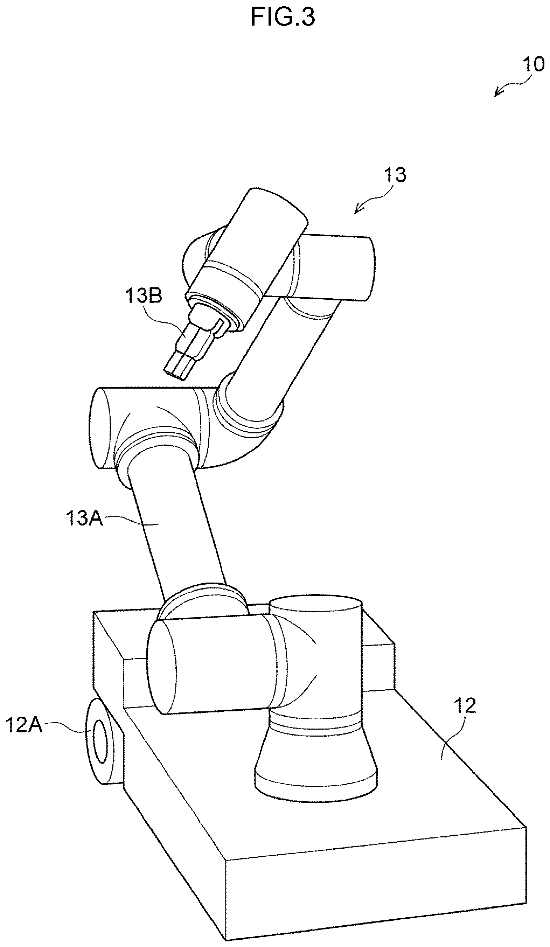

Fig. 3 is an external perspective view of a mobile manipulator.

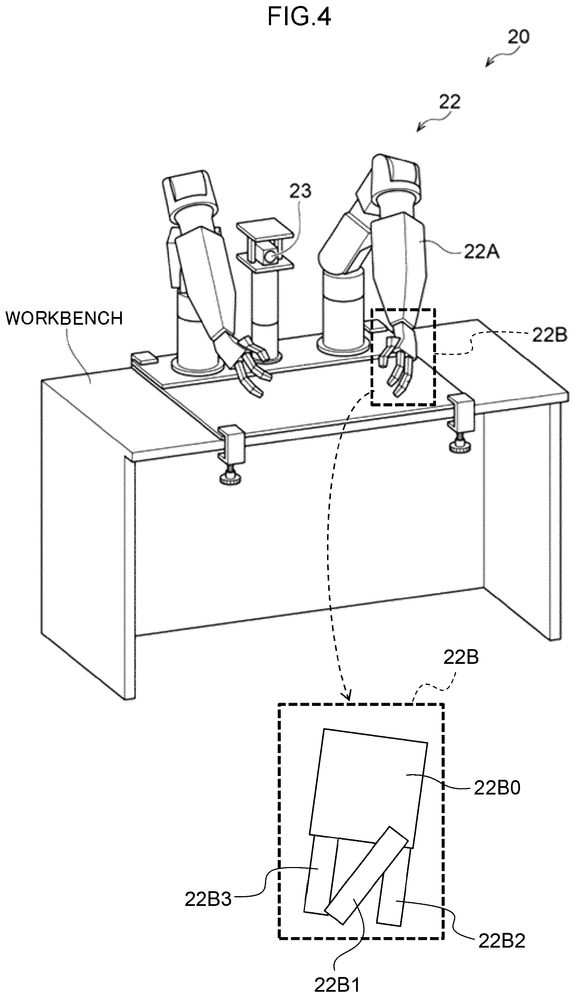

Fig. 4 is an external perspective view of a workbench robot.

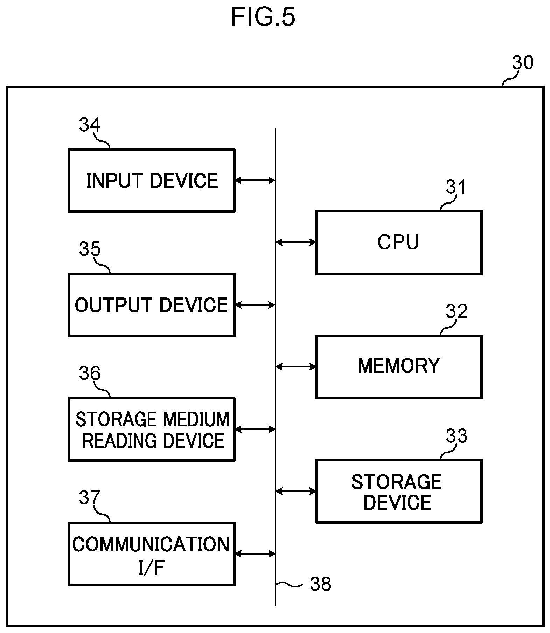

Fig. 5 is a block diagram showing the hardware configuration of an integrated controller.

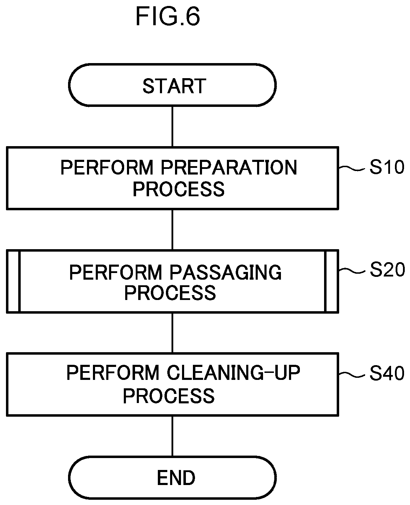

Fig. 6 is a flowchart showing the flow of a control process.

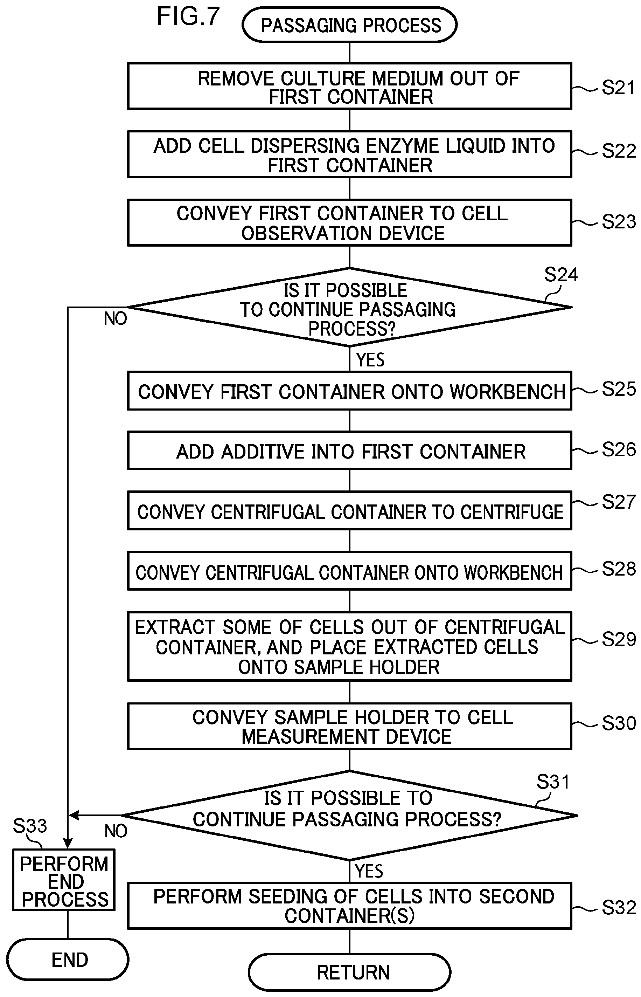

Fig. 7 is a flowchart showing the flow of a passaging process.

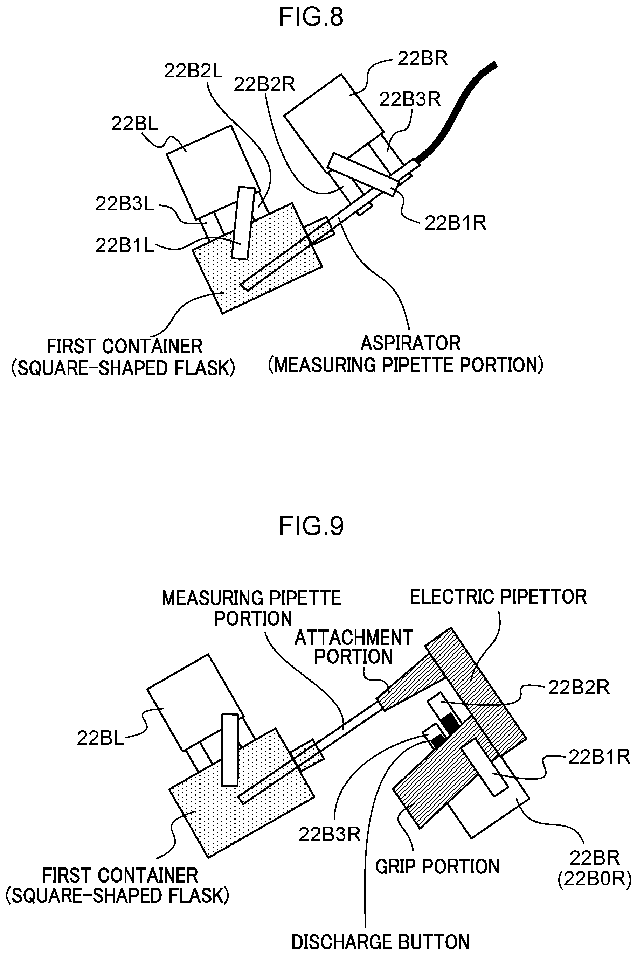

Fig. 8 is a diagram for explaining removal of a culture medium in a first container.
Fig. 9 is a diagram for explaining addition of a cell dispersing enzyme liquid into the first container.

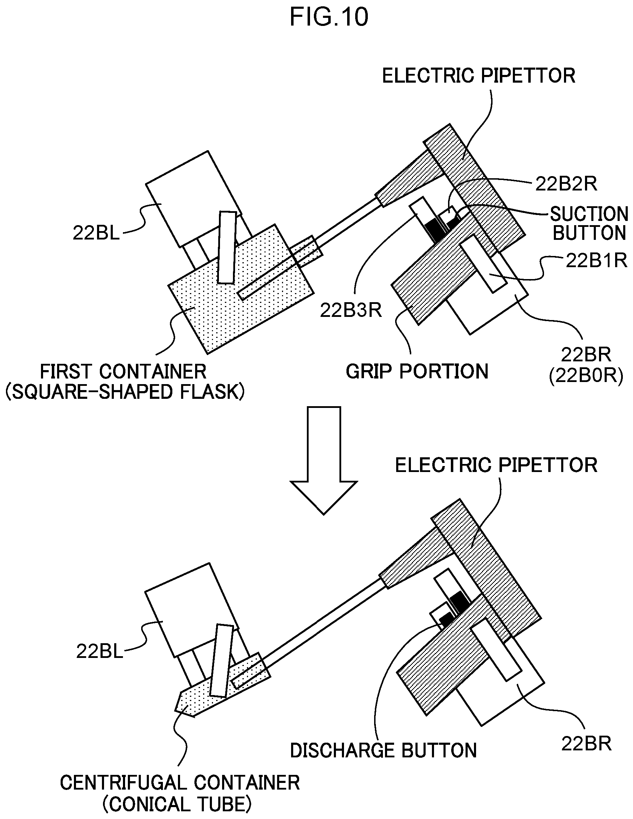

Fig. 10 is a diagram for explaining transfer of the mixture in the first container to a centrifugal container.

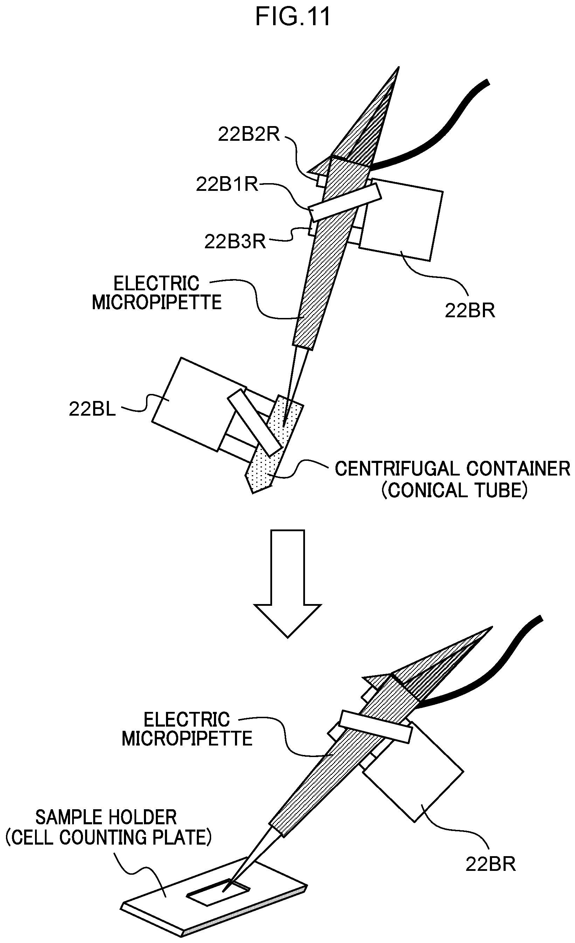

Fig. 11 is a diagram for explaining placement of some of the cells in the centrifugal container onto a sample holder.

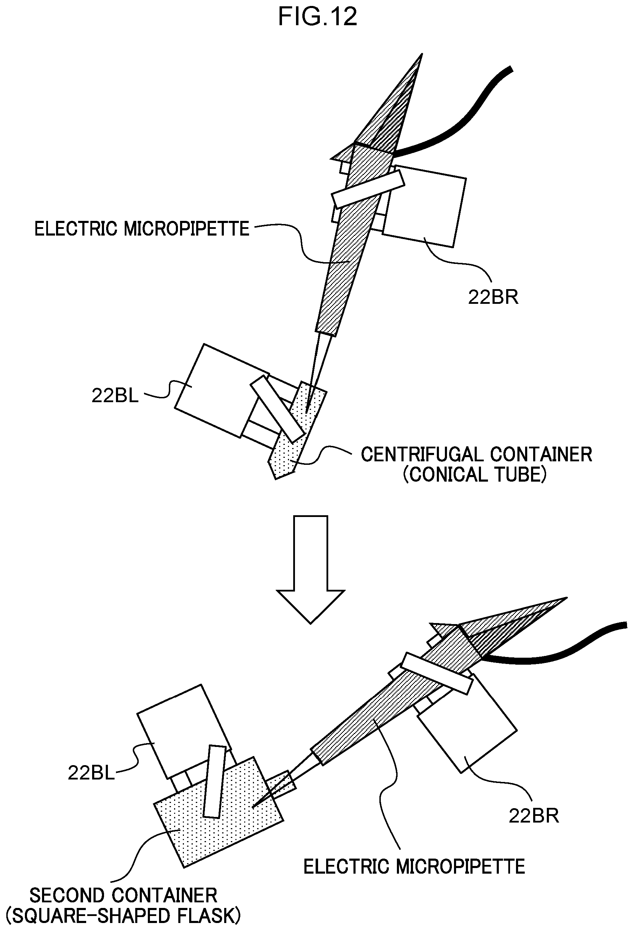

Fig. 12 is a diagram for explaining seeding of cells into a second container.

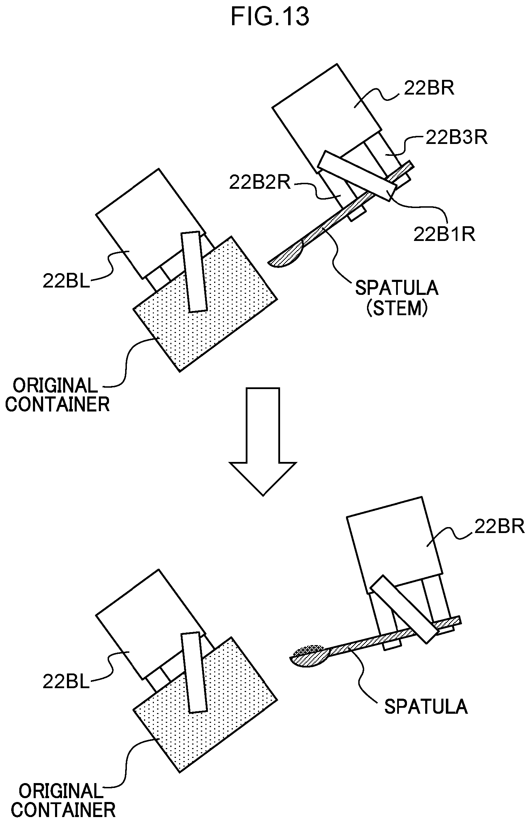

Fig. 13 is a diagram for explaining dispensing work.

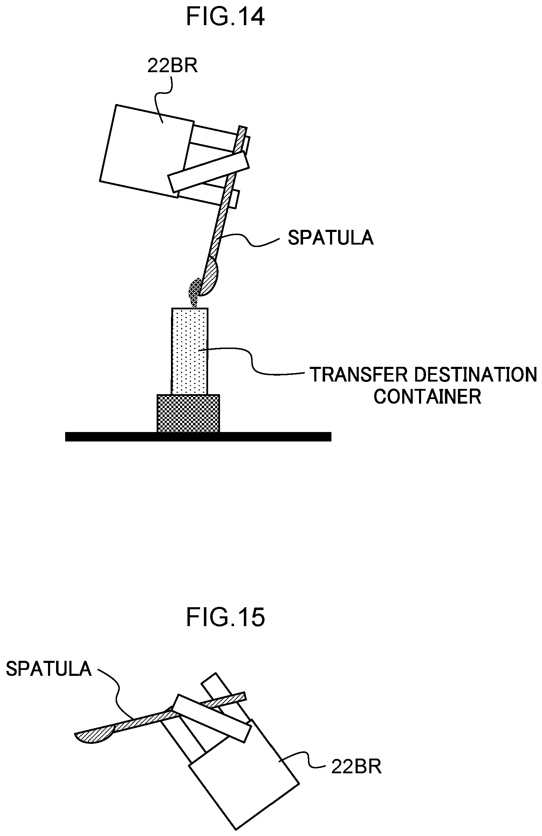

Fig. 14 is a diagram for explaining dispensing work.
Fig. 15 is a diagram for explaining another example of a manner of holding a spatula.

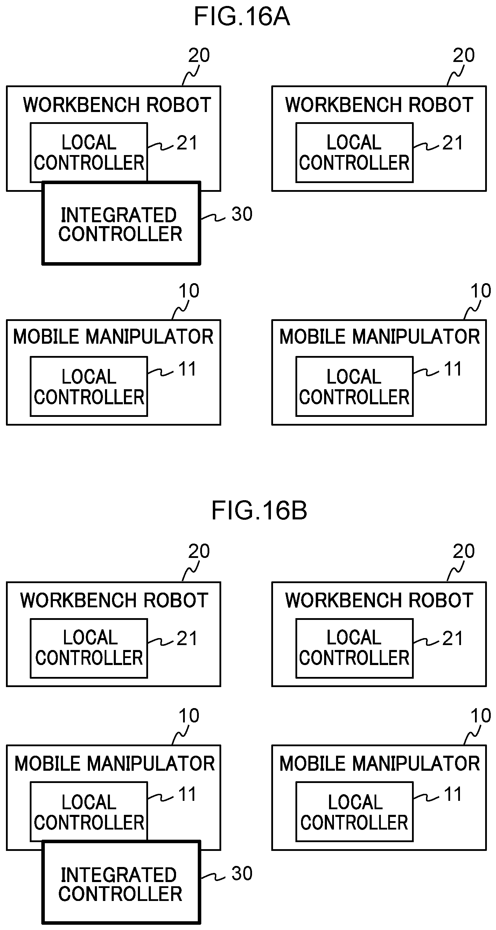

Fig. 16A is a diagram for explaining another example of the configuration of controllers.
Fig. 16B is a diagram for explaining another example of the configuration of controllers.

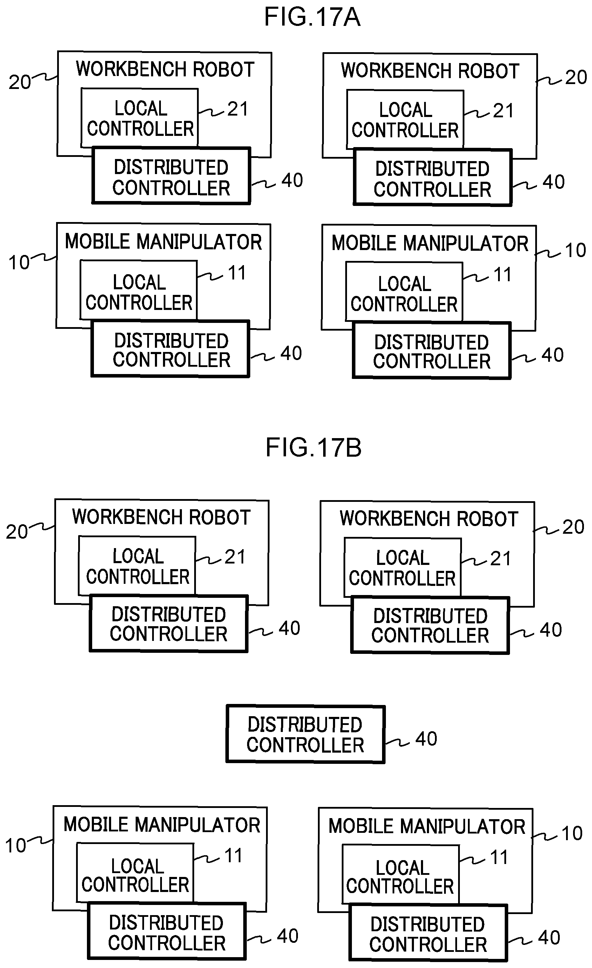

Fig. 17A is a diagram for explaining another example of the configuration of controllers.
Fig. 17B is a diagram for explaining another example of the configuration of controllers.

### DESCRIPTION OF EMBODIMENTS

In the following, an example of an embodiment of the present disclosure is described, with reference to the drawings. Note that, in the respective drawings, the same or equivalent constituent elements and components are denoted by the same reference signs. Further, the dimensions and ratios in the drawings are exaggerated for convenience sake, and might be different from actual ratios.

A typical example of an experimental environment that is used by researchers is an environment in which workbenches for performing most of the passaging work is installed, and a cell observation device, a cell measurement device, a centrifuge, and the like are installed at locations away from the workbenches. In a case where a researcher performs the passaging work, while the researcher performs the passaging work at a workbench, he/she, who is holding a sample container, walks and moves between the workbench and the cell observation device, the cell measurement device, or the centrifuge as necessary during the passaging work, to use these devices.

A robot system according to the present embodiment operates in an environment similar to the typical example of an experimental environment that is used by researchers. Fig. 1 is a schematic plan view of an example of an experimental environment in which the robot system according to the present embodiment operates. In the example in Fig. 1, in addition to the cell observation device, the cell measurement device, the centrifuge, and the workbenches described above, experimental facilities such as medicine cabinets, an incubator, a refrigerator, supplies cupboards, a charging station, a sink, and chairs are included in the experimental environment. Note that the microscope in Fig. 1 is an example of the above-described cell observation device and cell measurement device.

As illustrated in Fig. 2, a robot system 100 according to the present embodiment includes mobile manipulators 10, workbench robots 20, and an integrated controller 30. Note that, in the example in Fig. 2, two mobile manipulators 10 and two workbench robots 20 are shown, but it is not limited to this. The number of the mobile manipulators 10 and the number of the workbench robots 20 may be one, or may be three or larger.

A mobile manipulator 10 includes a local controller 21, a moving mechanism for moving on the floor, and a holding mechanism for gripping an object. Fig. 3 is an external perspective view of the mobile manipulator 10. As illustrated in Fig. 3, the mobile manipulator 10 includes a wheeled platform 12 and a robot arm 13.

The wheeled platform 12 is an example of the moving mechanism, and has two drive wheels 12A that are driven under the control of a local controller 11. Further, the wheeled platform 12 has a caster that changes freely in direction on the bottom surface on the front side in Fig. 3. Because the two drive wheels 12A are driven independently of each other, the wheeled platform 12 can move freely on the floor like the researchers. The drive mechanism is not limited to the two independent drive wheels, but may be any mechanism capable of moving on the floor, and is preferably a mechanism with fewer restrictions on the modes of movement including turning. The robot arm 13 is an example of the holding mechanism, and is mounted on the wheeled platform 12. The robot arm 13 includes an arm 13A and a hand 13B attached to the end of the arm 13A. The arm 13Amay be a vertical articulated arm having a configuration for displacing the position and posture of the hand 13B in, for example, six degrees of freedom in a three-dimensional space. The hand 13B is, for example, a two-finger robot hand capable of holding a container or the like. Note that Fig. 3 shows an example in which the mobile manipulator 10 includes one robot arm 13, but may be a two-arm manipulator including two robot arms 13.

The mobile manipulator 10 includes a vision sensor (not shown) at a position close to the hand 13B of the arm 13A. The vision sensor is a sensor for recognizing an object around the mobile manipulator 10 and a holding target such as a container to be held with the hand 13B. The vision sensor includes a camera and an image processing device, and recognizes an object in the surroundings and the holding target by performing, with the image processing device, image processing on an image captured by the camera. This enables autonomous movement of the mobile manipulator 10. Note that the sensor for recognizing objects in the surroundings and the holding target is not necessarily a vision sensor, and a laser radar or the like capable of measuring the three-dimensional position of each point in the periphery may be used. Also, the sensor for recognizing objects in the surroundings may be mounted on the wheeled platform 12.

The local controller 11 controls the operation of each motor of the mobile manipulator 10 so that instructions from the integrated controller 30 can be executed. Specifically, the local controller 11 controls the operation of the mobile manipulator 10 on the basis of a recognition result output from the vision sensor 14, an instruction from the integrated controller 30, or the like. More specifically, the local controller 11 controls the operation of the mobile manipulator 10 so that the holding target such as a container is held by the hand 13B, and the held holding target is conveyed to a predetermined position such as a workbench.

A workbench robot 20 is fixed to a workbench, and includes a local controller 21 and a holding mechanism for holding an object on the workbench. In addition to a container, the targets to be held by the workbench robot 20 includes an aspirator, an electric pipettor, and an electric micropipette for sucking cells or the like in the container and discharging cells or the like into the container. Fig. 4 shows an external perspective view of the workbench robot 20. As shown in Fig. 4, the workbench robot 20 includes two robot arms 22 and a vision sensor 23.

The robot arms 22 are an example of the holding mechanism, and each include an arm 22A and a hand 22B attached to the end of the arm 22A. The arm 22A may be a vertical articulated arm having a configuration for displacing the position and posture of the hand 22B in, for example, six degrees of freedom in a three-dimensional space. The hand 22B is, for example, a three-finger robot hand that is capable of holding a container or the like, each finger having a joint.

A schematic enlarged view of the hand 22B is shown in a lower portion of Fig. 4. In this schematic view, the joints of the respective fingers of the hand 22B are not illustrated, but the shapes and arrangement of the respective fingers are illustrated in a simplified manner. The hand 22B includes a first group of fingers including a first finger 22B1, a second group of fingers including a second finger 22B2 and a third finger 22B3, and a hand main body 22B0 to which the respective bases of the first finger 22B1, the second finger 22B2, and the third finger 22B3 are connected. In the hand 22B, the first group and the second group face each other, and can hold an object therebetween. As for the functions, the first finger 22B 1 corresponds to the thumb of a human hand, the second finger 22B2 corresponds to the index finger, and the third finger 22B3 corresponds to the middle finger. Note that the hand 22B may be a robot hand having four or more fingers. That is, the first group may include a finger in addition to the first finger 22B 1, and the second group may include a finger in addition to the second finger 22B2 and the third finger 22B3. Also, each finger of the hand 22B preferably has two joints in the middle. With this arrangement, it is possible to stably hold an object so as to wrap the outer shape of the object, and it is also easy to perform complicated operations such as pressing a button on a laboratory instrument.

As for the hands 22B of the two robot arms 22, in a case where the left hand and the right hand are distinguished from each other in the description below, the left hand will be referred to as the hand 22BL, and the right hand will be referred to as the hand 22BR. Likewise, as for the fingers of the hands 22B, the fingers of the left hand 22BL will be referred to as the first finger 22B1L, the second finger 22B2L, and the third finger 22B3L, and the fingers of the right hand 22BR will be referred to as the first finger 22B1R, the second finger 22B2R, and the third finger 22B3R. Also, as for the hand main bodies 22B0, the left hand main body will be referred to as the hand main body 22B0L, and the right hand main body will be referred to as the hand main body 22B0R.

The vision sensor 23 is a sensor that is mounted on a pan-tilt table and recognizes a holding target object such as a container placed on the workbench.

The local controller 21 controls the operation of each motor of the workbench robot 20 so that instructions from the integrated controller 30 can be executed. Specifically, the local controller 21 controls the operation of the workbench robot 20 on the basis of a recognition result output from the vision sensor 23, an instruction from the integrated controller 30, or the like. More specifically, the local controller 21 controls the operation of the workbench robot 20 so that the two robot arms 22 cooperate to execute a cell passaging process. For example, the local controller 21 controls the operation of the workbench robot 20 so as to execute processing such as holding a container with the hand 22B of one robot arm 22, holding a pipette with the hand 22B of the other robot arm 22, and sucking the liquid in the container.

Although Fig. 4 illustrates an example in which the workbench robot 20 is fixed directly to the workbench, the workbench robot 20 may be fixed to the floor, a wall, the ceiling, or the like, as long as a relative positional relationship with the workbench is secured.

The integrated controller 30 controls the operation of each of the mobile manipulators 10 and the workbench robots 20, in cooperation with the local controllers 11 of the mobile manipulators 10 and the local controllers 21 of the workbench robots 20. Specifically, the integrated controller 30 causes each of the mobile manipulators 10 and the workbench robots 20 to execute a cell passaging process including conveyance and installation of a container containing cells between a workbench and another experimental facility.

A holding operation and an operation on an experimental facility by a mobile manipulator 10 are conducted by the local controller 11 causing the mobile manipulator 10 to execute an operation plan taught in advance on the basis of a recognition result output from the vision sensor. The same applies to a holding operation and an operation on an experimental facility by a workbench robot 20.

Also, movement of a mobile manipulator 10 is conducted by the local controller 11 moving the mobile manipulator 10 to the position of an experimental facility designated by the integrated controller 30, on the basis of a layout diagram of the experimental environment stored in advance. Note that the local controller 11 moves the mobile manipulator 10 so as to move to a target position while avoiding obstacles, on the basis of a recognition result output from the vision sensor 14.

Fig. 5 is a block diagram showing the hardware configuration of the integrated controller 30. As shown in Fig. 5, the integrated controller 30 includes a central processing unit (CPU) 31, a memory 32, a storage device 33, an input device 34, an output device 35, a storage medium reading device 36, and a communication interface (I/F) 37. The respective components are communicably connected to one another via a bus 38.

The storage device 33 stores a program for performing the control process to be described later. The CPU 31 is a central arithmetic processing unit, and executes various programs and controls the respective components. That is, the CPU 31 reads a program from the storage device 33, and executes the program, using the memory 32 as a work area. The CPU 31 performs control on each of the above-described components and various kinds of arithmetic processing, in accordance with a program stored in the storage device 33.

The memory 32 is formed with a random access memory (RAM), and, as a work area, temporarily stores a program and data. The storage device 33 is formed with a read only memory (ROM), a hard disk drive (HDD), a solid state drive (SSD), and the like, and stores various programs including an operating system, and various kinds data.

The input device 34 is a device for performing various inputs, such as a keyboard and a mouse. The output device 35 is a device for outputting various kinds of information, such as a display and a printer. A touch panel display may be adopted as the output device 35, and be made to function also as the input device 34.

The storage medium reading device 36 reads data stored in various storage media such as a compact disc (CD)-ROM, a digital versatile disc (DVD)-ROM, a Blu-ray Disc, and a universal serial bus (USB) memory, and writes data into a storage medium, for example. The communication I/F 37 is an interface for communicating with other devices, and uses a standard such as Ethernet (registered trademark), FDDI, and Wi-Fi (registered trademark). Note that the other devices include the local controllers 11 of the mobile manipulators 10, the local controllers 21 of the workbench robots 20, a cell observation device, a cell measurement device, and a centrifuge. In the present embodiment, it is assumed that these other devices also have communication functions.

Note that the hardware configurations of the local controllers 11 of the mobile manipulators 10 and the local controllers 21 of the workbench robots 20 are substantially the same as the hardware configuration of the integrated controller 30, and therefore, explanation thereof is not made herein.

Next, the functions of the robot system 100 according to the present embodiment are described.

Fig. 6 is a flowchart showing a flow of a control process to be performed by the CPU 31 of the integrated controller 30. In the following, an example of adhesion culture is described.

In step S10, as a preparatory process toward a passaging process, the integrated controller 30 causes a mobile manipulator 10 to take out, from a first incubator, a first container containing the cells being cultured, and convey the first container onto a workbench. The first container is a square-shaped flask or the like.

Specifically, the integrated controller 30 instructs the local controller 11 of the mobile manipulator 10 to perform the preparatory process. Under the control of the local controller 11 that has received the instruction regarding the preparatory process, the mobile manipulator 10 moves to the position of the first incubator, opens the door of the first incubator, and holds the first container with the hand 13B. The mobile manipulator 10 then moves to the workbench while holding the first container, and places the first container onto the workbench.

Successively, in step S20, the integrated controller 30 causes the mobile manipulator 10 and the workbench robot 20 to perform the passaging process. Referring now to Fig. 7, the passaging process is described.

In step S21, the integrated controller 30 instructs the local controller 21 of the workbench robot 20 to remove the culture medium out of the first container. For example, as shown in Fig. 8, the local controller 21 having received the instruction controls the workbench robot 20 to hold the first container with the hand 22BL, hold the aspirator with the hand 22BR, and operate the aspirator to aspirate and remove the culture medium in the first container. More specifically, the local controller 21 controls the workbench robot 20 so that the first finger 22B1L of the hand 22BL and the second finger 22B2L and the third finger 22B3L face each other and hold the first container therebetween.

Also, the local controller 21 controls the workbench robot 20 so that the first finger 22B1R (the first group) of the hand 22BR and the second finger 22B2R and the third finger 22B3R (the second group) face each other and hold the aspirator therebetween. At this point of time, the local controller 21 puts the tip of the aspirator between the first group and the second group so that both the second finger 22B2R and the third finger 22B3R come into contact with the aspirator. This makes it possible to hold the aspirator more stably than in a case where the aspirator is held with only two fingers. In the case of holding with only two fingers, in a case of an external force is applied to the tip or the base side of the aspirator, the aspirator easily slips and rotates, with the held portion serving as the fulcrum. As described above, in the present embodiment, holding with three fingers makes it easier to maintain the held posture of the aspirator even in a case where an external force is applied to the aspirator.

Also, the local controller 21 may further cause the hand main body 22B0 to come into contact with the aspirator to hold the aspirator. In this manner, the held posture of the aspirator is further stabilized.

Note that the aspirator may be a tube that is flexible even at the end, or may be one in which a rigid pipe portion such as a measuring pipette is provided at an end of a flexible tube. In a case where a rigid pipe portion is provided, the entire hard pipe portion corresponds to the "vicinity of the tip of the aspirator".

The method of holding various containers with the respective fingers of a hand 22B in the following steps is substantially the same as the holding method in this step, and therefore, explanation thereof will not be repeated.

Successively, in step S22, the integrated controller 30 instructs the local controller 21 of the workbench robot 20 to add a cell dispersing enzyme liquid into the first container. The cell dispersing enzyme liquid is an example of a cell dispersing reagent, and is trypsin, for example. For example, as illustrated in Fig. 9, the local controller 21 having received the instruction controls the workbench robot 20 so that an electric pipettor is held with the hand 22BR, and the electric pipettor is operated to add the cell dispersing enzyme liquid into the first container.

The electric pipettor includes an attachment portion and a grip portion for a measuring pipette, and the grip portion includes a suction button and a discharge button. The suction button is a button that is pressed to issue an instruction regarding execution of an operation of sucking a liquid into the measuring pipette, and the discharge button is a button that is pressed to issue an instruction regarding execution of an operation of discharging a liquid from the measuring pipette. Therefore, as an operation of the electric pipettor, the local controller 21 has the grip portion of the electric pipettor sandwiched and held between the first finger 22B1R and the hand main body 22B0R. Note that the grip portion of the electric pipettor may be held also with portions other than the vicinity of the tips of the second finger 22B2R and the third finger 22B3R. The local controller 21 then causes either the second finger 22B2R or the third finger 22B3R (in the example in Fig. 9, the third finger 22B3R) to press the discharge button. Thus, the electric pipettor that cannot be handled with a two-finger hand can be operated.

Successively, in step S23, the integrated controller 30 instructs the local controller 11 of the mobile manipulator 10 to convey the first container to the cell observation device. The local controller 11 having received the instruction controls the mobile manipulator 10 so as to convey the first container having the cell dispersing enzyme liquid added thereto on the workbench to a cell observation device such as an optical microscope that performs enlarged image acquisition and image analysis of the observation target by computer control, and to place the first container at a predetermined position on the cell observation device. As the cell observation device, a device having an objective distance long enough to observe cells through a transparent wall surface of the first container is used.

Successively, in step S24, the integrated controller 30 determines whether the passaging process can be continued, on the basis of a result of the observation conducted by the cell observation device. For example, the integrated controller 30 acquires, from the cell observation device, an image or a result of an image analysis of the cells in the first container as an observation result, and, if the cells are sufficiently separated from the container wall and are in a floating state, determines that the passaging process can be continued. Note that the observation of the degree of cell detachment by the cell observation device may be automatically performed by image processing in the cell observation device or the integrated controller 30, or may be performed manually. If the passaging process can be continued, the process proceeds to step S25. If the passaging process cannot be continued, the process proceeds to step S33. Note that, instead of proceeding to step S33, observation by the cell observation device may be attempted again after awaiting for several minutes. This is because detachment of the cells from the container wall might progress with time in some cases.

Successively, in step S25, the integrated controller 30 instructs the local controller 11 of the mobile manipulator 10 to convey the first container onto the workbench. The local controller 11 having received the instruction controls the mobile manipulator 10 to take out the first container from the cell observation device, hold the first container, convey the first container to the workbench, and place the first container onto the workbench. Steps S23 to S25 described above may be omitted in a case where it can be estimated that the cells are sufficiently separated from the container wall, even without confirmation by the cell observation device. For example, such a case is a case where there is a record that the same kind of cells were cultured under the same conditions, and the cells were successfully separated from the container wall under the same conditions.

Successively, in step S26, the integrated controller 30 instructs the local controller 21 of the workbench robot 20 to add an additive, which is at least one of an enzyme reaction stopping liquid and a new culture medium, into the first container. For example, as illustrated in Fig. 9, the local controller 21 having received the instruction controls the workbench robot 20 so that the first container is held with the hand 22BL, and the electric pipettor is held with the hand 22BR to add the additive into the first container. Hereinafter, the first container containing the mixture of the cells and the additive, or a third container into which the contents of the first container have been transferred will be referred to as the centrifugal container.

In a case where the mixture of the cells and the additive is transferred into the third container, the integrated controller 30 instructs the local controller 21 of the workbench robot 20 to conduct the transfer. For example, as illustrated in the upper portion in Fig. 10, the local controller 21 having received the instruction controls the workbench robot 20 so as to operate the electric pipettor to suck the mixture out of the first container. Specifically, the local controller 21 has the grip portion of the electric pipettor sandwiched and held between the first finger 22B1R and the hand main body 22B0R. The local controller 21 then causes either the second finger 22B2R or the third finger 22B3R (in the example in the upper portion in Fig. 10, the second finger 22B2R) to press the suction button. Thus, the electric pipettor that cannot be handled with a two-finger hand can be operated.

As illustrated in the lower portion in Fig. 10, the local controller 21 then controls the workbench robot 20 so that the hand 22BL switches to holding the centrifugal container such as a conical tube. Further, the local controller 21 controls the workbench robot 20 to operate the electric pipettor to transfer the aspirated mixture to the centrifugal container. The operation of the electric pipettor herein is the same as that in the example in Fig. 9.

Note that, in a case where the first container is used as the centrifugal container as it is, the first container is not a square-shaped flask, but a conical tube is used from the beginning.

Successively, in step S27, the integrated controller 30 instructs the local controller 11 of the mobile manipulator 10 to convey the centrifugal container to the centrifuge. The local controller 11 having received the instruction controls the mobile manipulator 10 so as to convey the centrifugal container on the workbench to the centrifuge, and set the centrifugal container at a predetermined position (the rotating portion, for example) on the centrifuge. At this point of time, if necessary in terms of the specification of the centrifuge, the local controller 11 controls the mobile manipulator 10 to take out the member on which the centrifugal container is to be set from the centrifuge, set the centrifugal container on the member, and then return the member into the centrifuge. Further, the local controller 11 may control the mobile manipulator 10 to press a start button of the centrifuge to start an operation of the centrifuge. Note that the operation starting action for the centrifuge may be conducted by the integrated controller 30 transmitting a control signal to the centrifuge.

Successively, in step S28, the integrated controller 30 instructs the local controller 11 of the mobile manipulator 10 to convey the centrifugal container onto the workbench. The local controller 11 having received the instruction controls the mobile manipulator 10 to remove the centrifugal container out of the centrifuge, hold the centrifugal container, convey the centrifugal container to the workbench, and place the centrifugal container onto the workbench.

Successively, in step S29, the integrated controller 30 instructs the local controller 21 of the workbench robot 20 to extract some of the cells from the centrifugal container and place the extracted cells on a sample holder. The sample holder to be used herein is compatible with the specification of the cell measurement device. For example, in a case where the cell measurement device is an optical microscope, the sample holder is a glass slide. The glass slide may have a recess for receiving a sample. Depending on the specification of the cell measurement device, a certain container may be used as the sample holder.

For example, as illustrated in the upper portion in Fig. 11, the local controller 21 having received the instruction controls the workbench robot 20 so as to operate the electric micropipette to suck some of the cells out of the centrifugal container. As a result, the local controller 21 has the electric micropipette held between the first group (the first finger 22B1R) and the second group (the second finger 22B2R and the third finger 22B3R) so that both the second finger 22B2R and the third finger 22B3R are in contact with the electric micropipette. This makes it possible to hold the electric micropipette more stably than in a case where the electric micropipette is held with only two fingers. In the case of holding with only two fingers, in a case of an external force is applied to the tip or the base side (a cable, for example) of the electric micropipette, the electric micropipette easily slips and rotates, with the held portion serving as the fulcrum. As described above, in the present embodiment, holding with three fingers makes it easier to maintain the held posture of the electric micropipette even in a case where an external force is applied to the electric micropipette.

Also, the local controller 21 may have the electric micropipette held in such a manner that the hand main body 22B0 also comes into contact with the electric micropipette. This further stabilizes the held posture of the electric micropipette.

As illustrated in the lower portion in Fig. 11, the local controller 21 controls the workbench robot 20 to operate the electric micropipette to discharge the aspirated cells into the recess of the sample holder such as a cell counting plate.

Note that the electric micropipette is a device capable of sucking and discharging a small amount of cells. On-off control on suction and discharge by the electric micropipette may be wired electronic control via a USB cable or the like, and any switching on-off action by the hand 22B of the workbench robot 20 does not need to be performed. The integrated controller 30 may cause the workbench robot 20 to perform a process of staining the measurement target cells before cell measurement.

Successively, in step S30, the integrated controller 30 instructs the local controller 11 of the mobile manipulator 10 to convey the sample holder to the cell measurement device that measures the number of density of cells. The local controller 11 having received the instruction controls the mobile manipulator 10 so as to convey the sample holder on the workbench to the cell measurement device such as an optical microscope that performs enlarged image acquisition and image analysis of the observation target by computer control, for example, and to set the sample holder in an installation portion of the cell measurement device. Note that the "installation portion" herein refers to an installation site at which measurement by the cell measurement device is possible after the sample holder is set therein. The sample holder set in the installation portion may be moved to the measurement place by a function of the cell measurement device, and measurement is not necessarily possible with the sample holder remaining set in the installation portion.

Successively, in step S31, the integrated controller 30 determines whether the passaging process can be continued, on the basis of a result of the measurement performed by the cell measurement device. For example, the integrated controller 30 acquires, from the cell measurement device, the number or density of the cells in the sample holder as a measurement result, and, if the number of density of the cells is equal to or more than a predetermined threshold, determines that the passaging process can be continued. Note that the measurement of the number or density of cells by the cell measurement device may be performed automatically through image processing, or may be performed manually. If the passaging process can be continued, the process proceeds to step S32. If the passaging process cannot be continued, the process proceeds to step S33.

In step S32, the integrated controller 30 instructs the local controller 21 of the workbench robot 20 to perform seeding of the cells into one or a plurality of second containers. Note that the second container(s) may be similar to the first container. As for the second containers, there are a case where the size of the container is increased while the number of containers remains 1, and a case where the number of containers is increased. In the case of a container having a plurality of wells (recesses) formed therein, each of the wells is regarded as a second container.

For example, as illustrated in the upper portion in Fig. 12, the local controller 21 having received the instruction controls the workbench robot 20 so as to operate the electric micropipette to suck the amount of the cells (including the culture medium) for seeding into the second container(s) out of the centrifugal container. As illustrated in the lower portion in Fig. 12, the local controller 21 then controls the workbench robot 20 to operate the electric micropipette to discharge the aspirated cells into a second container such as a square-shaped flask. The local controller 21 repeats this control the same number of times as the number of second containers. Also, if necessary, the second container(s) may be replenished with the culture medium with the electric pipettor. The passaging process is then ended, and the process returns to Fig. 6 and proceeds to a cleaning-up process in step S40.

In step S33, the integrated controller 30 outputs from the output device 35 that the passaging process cannot be continued, and ends the passaging process and the control process. At that time, information indicating the reason why it is not possible to continue may be included in the output. In this manner, it becomes easier for a person who receives the output or the host system to grasp the situation of the passaging process and appropriately cope with the situation. Note that the control process may be continued for the next first container in the first incubator as a target of a passaging process. In this case, after step S33, the process may return to step S10 in the control process.

Successively, in step S40, the integrated controller 30 causes the mobile manipulator 10 to store the second container(s) containing the cells after the passaging process into a second incubator, as the cleaning-up process for the passaging process. The second incubator may be the same as the first incubator.

Specifically, the integrated controller 30 instructs the local controller 11 of the mobile manipulator 10 to perform the cleaning-up process. Under the control of the local controller 11 that has received the instruction regarding the cleaning-up process, the mobile manipulator 10 holds the second container on the workbench, moves to the position of the second incubator, opens the door of the second incubator, and stores the second container into the second incubator. The control process then comes to an end.

As described above, the local controller 21 causes the workbench robot 20 to perform the process of causing the hand 22B to hold the aspirator to aspirate the liquid, the process of causing the hand 22B to hold the electric pipettor to suck the liquid and the process of causing the electric pipettor to discharge the liquid, and the process of causing the hand 22B to hold the electric micropipette to suck the liquid and the process of causing the electric micropipette to discharge the liquid. In this manner, the series of works necessary for a cell passaging process in the workbench can be performed with one type of hand 22B.

Note that the workbench robot 20 can also be used for dispensing a medicine that is a powder material (a medicine before being converted into a solution), or, in other words, for transferring the medicine from the original container to the transfer destination container. The medicine dispensing work may be performed as preparatory work toward a passaging process or some other process. In the case of performing powder material dispensing work, the integrated controller 30 causes the local controller 21 of the workbench robot 20 to cause the workbench robot 20 to perform a process of causing the hand 22B to hold a spatula and scoop and transfer a powder material. Specifically, as illustrated in Fig. 13, the local controller 21 has the stem of the spatula held between the first group (the first finger 22B1R) and the second group (the second finger 22B2R and the third finger 22B3R) so that both the second finger 22B2R and the third finger 22B3R are in contact with the stem of the spatula. This makes it possible to hold the spatula more stably than in a case where the spatula is held with only two fingers. In the case of holding with only two fingers, in a case of an external force is applied to the tip (the scooping portion) of the spatula, the spatula easily slips and rotates, with the held portion serving as the fulcrum. As described above, in the present embodiment, holding with three fingers makes it easier to maintain the held posture of the spatula even in a case where an external force is applied to the spatula.

As illustrated in Fig. 13, the local controller 21 causes the workbench robot 20 to put the spatula into the original container containing the medicine (powder material), and scoop the medicine with the spatula. As illustrated in Fig. 14, the local controller 21 then causes the workbench robot 20 to drop the scooped medicine into the transfer destination container such as a conical tube that is erected on a stand or the like and is fixed thereto. This makes it possible to further facilitate the automation of the work on the workbench, including the dispensing work.

Although Figs. 13 and 14 illustrate an example in which the spatula is held with the hand 22B from above, the spatula may be held like a pen being held by a person, as illustrated in Fig. 15. Further, in a case where the transfer destination container is not fixed to a stand or the like, the hand 22BL may switch from holding the original container to holding the transfer destination container. Also, the hand main body 22B0 may be brought into contact with the stem of the spatula to hold the spatula. Further, in a case where the stem of the spatula has a plate-like shape, a flat portion (the front surface and the back surface) of the stem may be held, or side face portions of the stem may be held. For example, a lightweight holding target such as a spatula may be held with the two fingers of the first finger 22B1 and the second finger 22B2, or with the two fingers of the first finger 22B 1 and the third finger 22B3.

The controller 21 may cause the workbench robot 20 to perform at least two kinds of processes among the process of causing the hand 22B to hold an aspirator to aspirate a liquid, the process of causing the hand 22B to hold an electric pipettor to suck the liquid and the process of causing the electric pipettor to discharge the liquid, the process of causing the hand 22B to hold an electric micropipette to suck the liquid and the process of causing the electric micropipette to discharge the liquid, and the process of causing the hand 22B to hold a spatula to scoop and transfer a powder material. In this case, the number of types of work that can be performed with one type of hand can be increased in the robot system that performs a series of works on a workbench that requires handling of a plurality of types of tools used in the fields of experiments of handling biological samples such as cells, as compared with a case where a hand that is suitable for each form of the operation target is prepared, and the hand is replaced in response to a change in the operation target to be handled.

In the following, the robot system according to the present embodiment is supplementarily described using a cell passaging process as an example. The robot system according to the present embodiment includes a mobile manipulator that has a mechanism for moving on a floor and a mechanism for holding an object. Also, the robot system includes a workbench robot that is fixed to a workbench, and includes a mechanism for holding an object on the workbench. Further, the robot system includes a controller that controls the operation of each of the mobile manipulator and the workbench robot. The controller causes the workbench robot and the mobile manipulator to execute a cell passaging process including conveyance and installation of a container containing cells between a workbench and another experimental facility. With this arrangement, it is possible to operate in an environment similar to the experimental environment used by researchers, and automate the cell passaging process.

Specifically, in the robot system according to the present embodiment, the mobile manipulator not only conveys the cell container between an incubator and the workbench, but also intervenes in the course of the passaging process on the workbench. Thus, the passaging process can be automated in an experimental environment similar to that used by researchers.

For example, while the workbench robot can perform work at the desk in the same manner as a researcher, the mobile manipulator is responsible for the work that is performed in a case where the researcher is in charge of the passaging process, and in which the researcher walk with the container to reach the position of a microscope or a centrifuge. Thus, it is no longer necessary to create a dedicated robot environment in which devices and instruments that need to be present in the range in which the workbench robot can reach by hand are collected, and it is only required to form the desktop of the workbench into a dedicated robot environment. It is also possible to share the workbench with a researcher. Furthermore, since the workbench robot is a small robot of the size of the desktop of the workbench and can perform various works with one device instead of dedicated devices specialized for specific works, it is less necessary to prepare dedicated devices or a huge multifunctional device in the laboratory.

Although the case of adhesion culture has been described in the above embodiment, the robot system according to the present embodiment can also be adopted in the case of suspension culture. In this case, the integrated controller 30 causes the workbench robot 20 to extract some of the cell from the first container and place the cells onto a sample holder. The integrated controller 30 then causes the mobile manipulator 10 to convey the sample holder having the cells placed thereon to the cell measurement device that measures the number of cells or the cell density, and set the sample holder in the installation portion of the cell measurement device. In a case where the result of the measurement performed by the cell measurement device indicates that the passaging process can be continued, the integrated controller 30 causes the workbench robot to seed cells in one or a plurality of second containers.

Also, in the above embodiment, a case where the integrated controller 30 independent of the mobile manipulators 10 and the workbench robots 20 is used as an example of a controller of the present disclosure as illustrated in Fig. 2 has been described. However, the present disclosure is not limited to this. For example, as illustrated in Fig. 16(A), any one of the workbench robots 20 may be equipped with the integrated controller 30, or, as illustrated in Fig. 16(B), any one of the mobile manipulators 10 may be equipped with the integrated controller 30.

Also, as illustrated in Figs. 17(A) and 17(B), a controller of the present disclosure may include a plurality of distributed controllers 40. In this case, the distributed controllers communicate with each other to control the mobile manipulators 10 and the workbench robots 20 to operate in cooperation with each other. For example, in a case of a mobile manipulator 10 conveys a container onto a workbench, the distributed controller 40 of the mobile manipulator 10 notifies the distributed controller 40 of the workbench robot 20 that the conveyance has been completed. The distributed controller 40 that has received the notification then enables the workbench robot 20 to start processing on the conveyed container.

Note that the local controllers 11 and 21 are not necessarily provided in the respective mobile manipulators 10 and the respective workbench robots 20, and the integrated controller 30 or the distributed controllers 40 may control operations of the respective motors of the mobile manipulators 10 and the workbench robots 20.

Further, in addition to the above processes, each mobile manipulator 10 of the above embodiment may perform a process of conveying a container containing a medicine to be used in a cell passaging process from a refrigerator or a medicine cabinet onto a workbench. Also, each mobile manipulator 10 may perform a process of putting the container containing the remaining unused medicine into a refrigerator or a medicine cabinet. Further, each mobile manipulator 10 may perform a process of conveying a tool such as a pipette to be used in a cell passaging process, an empty container, and the like from a supplies cupboard onto a workbench. Also, each mobile manipulator 10 may move to a charging station to charge the mobile manipulator in a case of it is time for charging.

Further, in the above embodiment, the integrated controller 30 may check the operating status of each of the mobile manipulators 10 and the workbench robots 20, and cause a mobile manipulator 10 or a workbench robot 20 in an unbusy state to execute the above processes. Further, as for the mobile manipulators 10, position information in the experimental environment may be acquired, and the mobile manipulator 10 that is capable of most efficiently moving to a designated position may be selected from among the mobile manipulators 10 in an unbusy state, and be made to perform the above processes.

As for the workbench robots 20, even if the cells being processed on the workbench have been taken out to a centrifuge, for example, a series of processes may be performed by the same workbench robot 20 continuously using an instrument or the like on the workbench after the centrifugal process. However, centrifugation for a plurality of containers prepared on one workbench may be performed collectively, and aspiration may be performed in parallel by a plurality of workbench robots 20 so as to quickly perform subsequent aspiration (suction removal of the supernatant). In this manner, a plurality of the workbench robots 20 may be used in a passaging process of cells incubated in one container.

Although an optical microscope has been described as an example of the cell observation device in the above embodiment, the cell observation device is not limited to this, and any observation method and principles may be used as long as the device is capable of observing the state of the cells in accordance with the purpose, such as an observation device by a laser scanning method. Likewise, the cell measurement device may be any device capable of measuring the number of cells or the like in accordance with the purpose, regardless of the measurement method and principles, such as a measurement device by a laser scanning method. It is not essential that the measurement can be completed by the device, and, for example, there may be cases where the cell measurement device is a microscope, and a person visually counts the number of cells. The cell observation device and the cell measurement device may be one device that serves as both devices, or may be separate devices.

Note that the cell observation device typically observes the cells in the first container through the wall surface of the first container, and the observed cells are to be cultured thereafter. On the other hand, the cell measurement device typically measures a trace amount of extracted cells, and the measured cells are discarded.

Although a case where each mobile manipulator 10 conveys a container between the workbench to which a workbench robot 20 is fixed and the cell observation device, the cell measurement device, or the like has been described in the above embodiment, the present disclosure is not limited to this case. For example, the cell observation device, the cell measurement device, and the like may be installed on the workbench to which a workbench robot 20 is fixed, or in a range that is adjacent to the workbench and within which the hand 22B of the workbench robot 20 can reach. In this case, the workbench robot 20 may place and remove a container on and from the cell observation device, the cell measurement device, or the like.

Further, the robot system processes performed by the CPU reading software (a program) in the above embodiment may be performed by various processors other than a CPU. Examples of the processors in this case include a programmable logic device (PLD) in which the circuit configuration can be changed after manufacturing, such as a field-programmable gate array (FPGA), and a dedicated electric circuit that is a processor having a circuit configuration designed exclusively for performing specific processing, such as an application specific integrated circuit (ASIC). Also, the robot system processes may be performed by one of these various processors, or may be performed by a combination of two or more processors of the same type or different types (a plurality of FPGAs, a combination of a CPU and an FPGA, or the like). More specifically, the hardware structure of any of these various processors is an electric circuit in which circuit elements such as semiconductor elements are combined.

Although the aspect in which the robot system program is stored (installed) beforehand into a storage device has been described in the above embodiment, the disclosure is not limited to this. The program may be provided in a form stored in a storage medium such as a CD-ROM, a DVD-ROM, a Blu-ray Disc, or a USB memory. Alternatively, the program may be downloaded from an external device via a network.

### Reference Signs List

100 Robot system
10 Mobile manipulator
11 Local controller
12 Wheeled platform
12A Drive wheel
13 Robot arm
13A Arm
13B Hand
20 Workbench robot
21 Local controller
22 Robot arm
22A Arm
22B, 22BL, 22BR Hand
22B0, 22B0L, 22B0R Hand main body
22B1, 22B1L, 22B1R First finger
22B2, 22B2L, 22B2R Second finger
22B3, 22B3L, 22B3R Third finger
23 Vision sensor
30 Integrated controller
31 CPU
32 Memory
33 Storage device
34 Input device
35 Output device
36 Storage medium reading device
37 Communication I/F
38 Bus
40 Distributed controller

## Claims

1. A robot system comprising: a workbench robot including a manipulator that manipulates an object on a workbench; and a controller that controls an operation of the workbench robot, wherein:
the manipulator includes an articulated arm, and a hand that is positioned at a distal end of the arm and that includes a plurality of fingers having joints,
the hand has a structure in which a first group of fingers including a first finger and a second group of fingers including a second finger and a third finger face each other and hold the object between the first group and the second group, and
the controller causes the workbench robot to perform at least two kinds of processes among:
a process of causing the hand to hold an aspirator to aspirate a liquid,
a process of causing the hand to hold an electric pipettor to suck the liquid and a process of causing the electric pipettor to discharge the liquid,
a process of causing the hand to hold an electric micropipette to suck the liquid and a process of causing the electric micropipette to discharge the liquid, or
a process of causing the hand to hold a spatula to scoop and transfer a powder material.

2. The robot system according to claim 1, wherein the controller causes the workbench robot to perform at least:
the process of causing the hand to hold the aspirator to aspirate the liquid,
the process of causing the hand to hold the electric pipettor to suck the liquid and the process of causing the electric pipettor to discharge the liquid, and
the process of causing the hand to hold the electric micropipette to suck the liquid and the process of causing the electric micropipette to discharge the liquid.

3. The robot system according to claim 1 or 2, wherein, in a case of causing the workbench robot to perform the process of causing the hand to hold the aspirator to aspirate the liquid, the controller causes the hand to hold a vicinity of a tip of the aspirator between the first group and the second group in such a manner that both the second finger and the third finger are in contact with the aspirator.

4. The robot system according to claim 1 or 2, wherein:
the hand includes a hand main body to which a base of each of the first finger, the second finger, and the third finger is connected,
the electric pipettor includes an attachment portion and a grip portion of a measuring pipette, the grip portion including a suction button that is pressed to issue an instruction for execution of an operation of sucking the liquid into the measuring pipette and a discharge button that is pressed to issue an instruction for execution of an operation of discharging the liquid from the measuring pipette, and
the controller causes the first finger and the hand main body to sandwich and hold the grip portion of the electric pipettor in a case of causing the workbench robot to perform the process of causing the hand to hold the electric pipettor to suck the liquid and the process of causing the electric pipettor to discharge the liquid, and causes one of the second finger or the third finger to press the suction button in a case of causing the workbench robot to perform the process of causing the electric pipettor to suck the liquid, and causes another one of the second finger or the third finger to press the discharge button in a case of causing the workbench robot to perform the process of causing the electric pipettor to discharge the liquid.

5. The robot system according to claim 1 or 2, wherein, in a case of causing the workbench robot to perform the process of causing the hand to hold the electric micropipette to suck the liquid and the process of causing the electric micropipette to discharge the liquid, the controller causes the hand to hold the electric micropipette between the first group and the second group in such a manner that both the second finger and the third finger are in contact with the electric micropipette.

6. The robot system according to claim 1, wherein, in a case of causing the workbench robot to perform the process of causing the hand to hold the spatula to scoop and transfer the powder material, the controller causes the hand to hold a stem of the spatula between the first group and the second group in such a manner that both the second finger and the third finger are in contact with the stem of the spatula.
